(19) Europäisches Patentamt
European Patent Office
Office européen des brevets

(11) **EP 3 745 130 B1**

(12) **EUROPEAN PATENT SPECIFICATION**

(45) Date of publication and mention
of the grant of the patent:
**28.07.2021 Bulletin 2021/30**

(51) Int Cl.:
***G01N 33/543*** *(2006.01)*   ***G01N 33/553*** *(2006.01)*

(21) Application number: **20176719.1**

(22) Date of filing: **27.05.2020**

(54) **BIOSENSOR AND METHOD FOR THE EARLY DIAGNOSIS OF GLAUCOMA**

BIOSENSOR UND VERFAHREN ZUR FRÜHEN DIAGNOSE VON GLAUKOM

BIOCAPTEUR ET PROCÉDÉ DE DIAGNOSTIC PRÉCOCE DU GLAUCOME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.05.2019 IT 201900007725**

(43) Date of publication of application:
**02.12.2020 Bulletin 2020/49**

(73) Proprietor: **Eye Pharma S.p.A.**
**16148 Genova (GE) (IT)**

(72) Inventors:
• **Pulliero, Alessandra**
  **16166 Genova GE (IT)**
• **Saccà, Sergio Claudio**
  **16121 Genova GE (IT)**
• **Maggi, Norbert**
  **16148 Genova GE (IT)**

(74) Representative: **Longoni, Alessandra**
**AL & Partners Srl**
**Via C. Colombo ang. Via Appiani**
**(Corte del Colone)**
**20831 Seregno (MB) (IT)**

(56) References cited:
**RU-A- 2012 149 809**

• **A IZZOTTI ET AL: "Molecular Damage in Glaucoma: from Anterior to Posterior Eye Segment. The MicroRNA Role", MICRORNA, vol. 4, no. 1, 1 January 2015 (2015-01-01), pages 3-17, XP055658531,**
• **DATABASE BIOSIS [Online] BIOSCIENCES INFORMATION SERVICE, PHILADELPHIA, PA, US; 2003, IWATA T ET AL: "ANALYSIS OF OPTINEURIN - RAB8 PROTEIN INTERACTION UISNG QUARTZ - CRYSTAL MICROBALANCE ( QCM )", XP002796937, Database accession no. PREV200300512386 & ARVO ANNUAL MEETING ABSTRACT SEARCH AND PROGRAM PLANNER, vol. 2003, 2003, page Abstract No. 1114, ANNUAL MEETING OF THE ASSOCIATION FOR RESEARCH IN VISION AND OPHTHALMOLOGY; FORT LAUDERDALE, FL, USA; MAY 04-08, 2003**

**Description**

The present invention relates to an *in vitro* method for the early diagnosis of glaucoma based on the quantification of nestin in the aqueous humor of a patient and a functionalized biosensor that is particularly suitable for performing the method.

## BACKGROUND OF THE INVENTION

The term *"glaucoma"* refers to a group of ocular neurodegenerative disorders which represents the main cause of irreversible blindness worldwide whose incidence is constantly increasing. Glaucoma is characterized by altered aqueous humor outflow with a consequent increase of the intraocular pressure, by the excavation of the optic nerve head (ONH) and by the slow progressive loss of retinal ganglion cells (RGCs).

Examinations and tests such as ocular tonometry (measurement of the intraocular pressure), pachymetry (measurement of the central corneal thickness), gonioscopy (anterior chamber angle examination), perimetry (visual field test), stereo photography and/or optic nerve imaging (for example, OCT) are commonly used for the diagnosis of glaucoma. However, when the eye structures are damaged and therefore the glaucoma can be diagnosed, the vision is already irreversibly damaged.

It has been discovered rather recently that in glaucomatous patients some stimuli such as high levels of oxidative stress affect the gene expression of several cells, including the trabecular cells, resulting in significative changes of the proteomic profile of the aqueous humor. In particular, it has been found that nestin, a protein responsible for glial activation, is overexpressed in the aqueous humor of glaucomatous patients (see, for example, Izzotti et al., "Molecular Damage in Glaucoma: from Anterior to Posterior Eye Segment. The MicroRNA Role" (2015)). Since the concentration of nestin in the aqueous humor of patients affected by open angle glaucoma starts to increase before the appearance of the alterations of the eye structures and before the appearance of the symptoms, nestin could be advantageously used as biomarker of the disease. RU2012149809 discloses the marker Bcl-2 in lachrymal fluid for predicting glaucoma.

It is also known that the identification and/or quantification of nestin can occur according to several immunochemical techniques such as, for example, radioimmunoassay, immunofluorescence and immunoblotting. However, the use of these techniques is limited by the danger and short half-life of radioactive markers, by the high costs of the equipment, by the complexity of the procedures, by the long incubation times and by the fact that the interpretation of the results needs to be carried out by specialized personnel.

Therefore there is still the need for a method which does not have the drawbacks of the known methods of the art and allows an early diagnosis of glaucoma, before the eye structures being compromised and before the appearance of the symptoms of visual impairment.

The Inventors have now found an *in vitro* method for the early diagnosis of the open angle glaucoma based on the quantification of nestin in the aqueous humor of a patient.

## BRIEF DESCRIPTION OF THE FIGURES

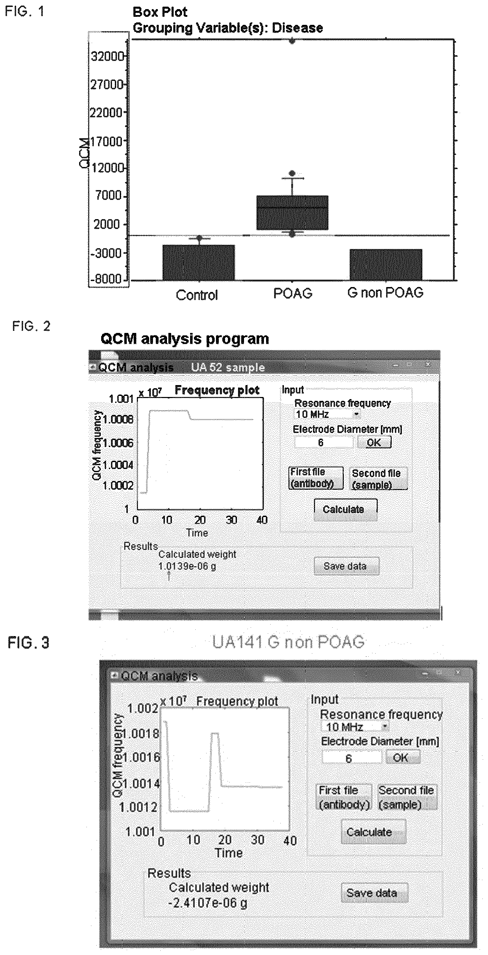

Figure 1: oscillation frequency plot for healthy subjects (control), patients suffering from primary open angle glaucoma (POAG), patients suffering from non open angle glaucoma (G non POAG). The threshold value (dashed line) is about 1000 Hz.

Figure 2: oscillation frequency vs time plot for an aqueous humor sample of a patient suffering from POAG. In figure 2 it is shown the QCM read of a sample related to a patient with POAG glaucoma at a resonance frequency of 10MHz. The calculation of the weight of the sample by the Sauerbrey formula, already entered the software, reported at the right bottom determines the value in nanograms of the nestin protein which is present.

Figure 3: oscillation frequency vs time plot for an aqueous humor sample of a patient suffering from glaucoma non POAG. In figure 2 it is shown the QCM read of a sample related to a patient with POAG glaucoma at a resonance frequency of 10MHz. The calculation of the weight of the sample by the Sauerbrey formula, already entered into the software, reported at the right bottom determines the value in nanograms of the nestin protein which is present. In this case the value is negative since the subject is a healthy subject.

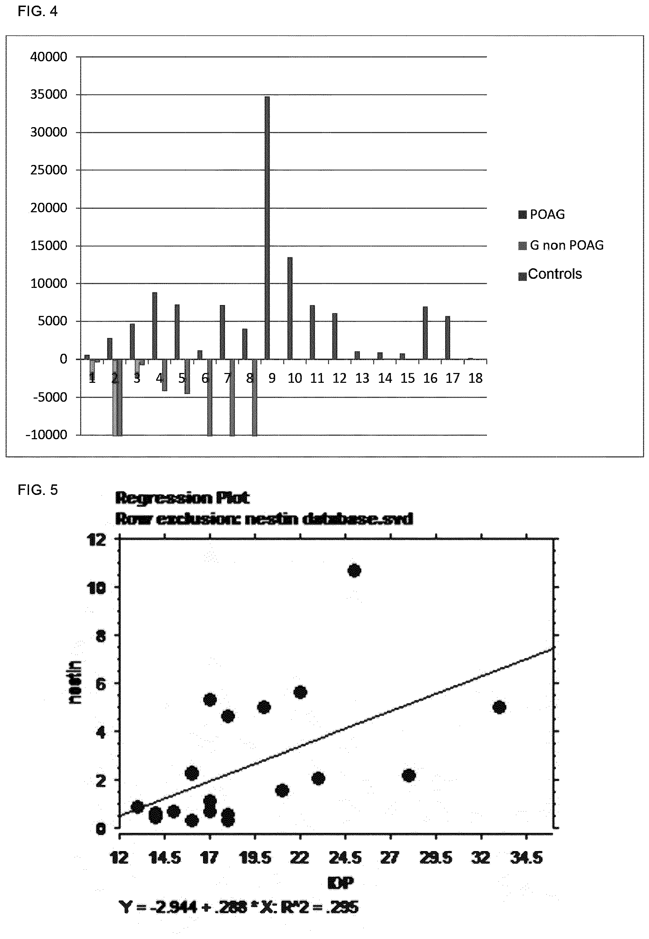

Figure 4: the results of 29 subjects analyzed by QCM are reported. It can be seen that all the values in nanograms reported in black are positive since they are referred to patients with overt POAG disease. The healthy subjects are reported in dark grey, with negative nestin values, because nestin is not present. On the contrary, the subjects having the disease, wherein the disease is not open angle glaucoma but another form of glaucoma, are reported in light grey. They show negative nestin values so excluding the presence of the protein.

Figure 5: correlation plot between IOP and nestin amount in patients with POAG.

DETAILED DESCRIPTION OF THE INVENTION

After extensive experimentation, the inventors of the present invention have now surprisingly developed a method for the early diagnosis and/or prognosis of glaucoma which is based on the quantification of nestin in the aqueous humor of a patient and is able to overcome the drawbacks of the known methods.

Therefore, object of the present invention is a method for the early diagnosis of glaucoma comprising the determination of the amount of nestin in an aqueous humor sample of a patient by using a biosensor functionalized with an anti-nestin antibody and a quartz crystal microbalance.

In the present context, the term "glaucoma" refers to primary open angle glaucoma (POAG).

The term "early diagnosis" means a diagnosis carried out before the appearance of the symptoms of visual impairment, that is before the appearance of the clinical symptoms of the disease.

As used herein, the term "nestin" refers to the human protein nestin, also referred to as NES, well known in the literature (reference sequence NM_006617.2 obtained from the databank of the National Center for Biotechnology Information, NCBI).

The term "anti-nestin antibody" refers to a specific human monoclonal antibody, which directly binds to the nestin molecule.

The method object of the present invention is based on the use of a biosensor functionalized with an anti-nestin antibody and on the determination of the amount of nestin in an aqueous humor sample of a patient by using a quartz crystal microbalance.

The functionalized biosensor useful in the method object of the present invention can be a biosensor made of any metal material, functionalized with anti-nestin antibodies according to any immobilization techniques known in the art. Said functionalized biosensor will be suitably shaped and sized to be placed on the suitable compartment of the quartz crystal microbalance.

However, the inventors of the present invention have developed a specific functionalized biosensor, particularly suitable to the use in the method for the early diagnosis of glaucoma object of the present invention.

Said functionalized biosensor, which is a further object of the present invention, comprises a gold electrode, a self-assembling layer and an identification layer, said identification layer comprising anti-nestin antibodies.

The self-assembling layer comprises bifunctional molecules able to bind to the electron's gold and to be functionalized to covalently bind the anti-nestin antibody. Particularly suitable bifunctional molecules are molecules containing two functional groups selected among a thiol group (-SH) and a carboxy (-COOH) or hydroxy (-OH) group, preferably separated by a $C_6$-$C_{18}$, still more preferably $C_8$-$C_{12}$, linear hydrocarbon chain. Particularly preferred specific examples of said molecules are mercaptoundecanoic acid and mercaptoundecanol, which are still more preferably used in admixture each other. Instead, the identification layer comprises anti-nestin antibodies.

Preferably the functionalized biosensor has a diameter from 6 to 14 mm.

The functionalized biosensor can be disposable or multi-use, preferably disposable. The functionalized biosensor is preferably sterile and packaged within a case able to preserve its sterility up to the time of use, for example, within a plastic case with a strip closure system.

Any known quartz crystal microbalance (QCM) can be used in the method object of the present invention. For example, the Novatech openQCM Q$^{-1}$ or openQCM Wi2 models can be used.

A QCM typically consists of a thin quartz disk equipped with a functionalized electrode able to bind a specific analyte. Since the oscillation frequency is proportional to the mass deposited on the functionalized electrode, QCM is able to provide qualitative and quantitative measures related to the presence of the analyte. In the method of the present invention, a biosensor is functionalized with an anti-nestin antibody and placed on a piezoelectric quartz crystal (nominal frequency = 10 MHz) in a microbalance. The application of alternating current results in the mechanical oscillation of the quartz disk and the balance measures the oscillation frequency of the crystal in the presence of the functionalized biosensor (control). When the functionalized biosensor is exposed to an aqueous humor sample containing nestin (the antigen selectively reacting with the antibody bound to the surface of the functionalized biosensor) an antigen-antibody complex is formed resulting in a mass change on the crystal surface which, in its turn, results in an oscillation frequency change.

In a preferred embodiment, the method of the present invention comprises the following steps:

i. measuring the oscillation frequency of the quartz crystal by quartz crystal microbalance in the presence of a biosensor functionalized with anti-nestin antibodies;
ii. adding an aqueous humor sample on said biosensor;
iii. measuring the change of oscillation frequency of the quartz crystal; and
iv. determining the amount of nestin in the aqueous humor sample.

Once the change of oscillation frequency is measured, the determination of the amount of nestin as weight or concentration (by weight or by volume) is carried out on the basis of the Sauerbrey equation herein after reported:

$$\Delta f = -\frac{2f_0^2}{A\sqrt{\rho_q \mu_q}}\Delta m$$

wherein $f_0$ is the oscillation frequency, $\Delta f$ the frequency change, $\Delta m$ the mass change, $A$ the active area of the crystal, $\rho_q$ the quartz density ($\rho_q$ = 2.648 g/cm$^3$), $\mu_q$ quartz distortion module ($\mu_q$ = 2.947 x 1011 g·cm$^{-1}$·s$^{-2}$).

Since $f_0$, $A$, $\rho_q$ and $\mu_q$ are constant, the equation can be also represented as:

$$\Delta f = -C_f \Delta m$$

The weight changes of nestin can be therefore substantially and very accurately (up to 10 ng cm$^{-2}$) calculated from the frequency changes.

The presence or absence of nestin in the analyzed aqueous humor sample allows to diagnose the disease or to exclude that the patient is affected by the disease.

There is also a linear correlation between the value of nestin and the progress stage of the disease: the higher the value of the amount of nestin, the more serious the disease.

Reference values correlating the amount of nestin to a disease severity index can be calculated, by using the method of the present invention, in the aqueous humor of samples from patients surely suffering from glaucoma.

Therefore, the diagnostic method of the present invention can be further used for the prognosis of a patient suffering from glaucoma.

In a preferred embodiment of the method of the present invention, the correlation between the amount of nestin determined in the aqueous humor of the patient and the reference values used as index of the stage of the disease is carried out automatically by a dedicated software which, when implemented in an electronic device equipped with a detector, is able to convert the frequency change into mass change by using the Sauerbrey equation, to compare the resulting value with the reference values and then to make sure that the detector emits a light signal of a different color depending on the presence or absence of nestin. As an example, said light signal can be red when the presence of nestin is detected (patient with glaucoma) and green when nestin is absent (healthy subject). The software is also able to save the obtained data, expressed as calculated weight of nestin in the aqueous humor of the patient so they can be subsequently displayed and analyzed.

The advantages deriving from the method of the present invention are promptly evident. In particular, the method of the present invention is ease, fast, repeatable, specific and sensitive, does not require the labeling of the analytes (label-free detecting system) or the intervention of specilized personnel for the interpretation of the results, provides an objective result, allows the prompt quantification of nestin and, above all, the diagnosis of the disease before the appearance of visual impairments.

A further object of the present invention is a kit for the early diagnosis of glaucoma comprising the functionalized biosensor of the present invention contained into a device with a plastic case and an USB port allowing the connection to a PC for the recording and management of the detected data.

Some examples are now provided to illustrate in details, without limiting it, how to practice the method object of the present invention.

Example 1

Preparation and use of the functionalized biosensor

The functionalization was carried out on a biosensor consisting of a piezoelectric crystal and a gold electrode.

The biosensor was incubated for 12 hours with a mixture of 11-mercapto-1-undecanoic acid (4mM) and 11-mercaptoundecanol (1nM), so that the thiol groups reacted with the gold of the electrode and attached to it to give a self-assembling layer. After the incubation, the self-assembling layer was washed with ethanol to remove the unbound thiol groups. Subsequently, in order to activate the self-assembling layer, the biosensor was incubated for 10 minutes with a 1:1 mixture in volume of N-hydroxysuccinimide (NHS - 100mM) and 1-ethyl-3-[3-dimethylaminopropyl]carbodimide hydrochloride (EDC - 400 mM) in millipore water.

The excess of NHS/EDC complex was removed with PBS (phosphate-buffered saline). Then, in order to immobilize the antibody on the self-assembling layer and form the identification layer, the biosensor was incubated with a 1 mg/ml solution (1:100 dilution) of anti-nestin antibody for about 30 minutes. A washing with PBS was then carried out and 1M ethanolamine hydrochloride (pH = 8.5) was added for 7 minutes, so closing the unreacted sites. After this washing 1M ethanolamine hydrochloride (100 μL) was added to block the active sites available to binding primary amines and after 15 minutes the frequency value was recorded by QCM. Then a washing with PBS was carried out and the functionalized biosensor was contacted with an aqueous humor sample (30 μL) for 15 minutes. The frequency value was then recorded by QCM and the amount of nestin in the sample calculated from the recorded frequency change.

Example 2

Determination of the amount of nestin in aqueous humor samples from patients

Aqueous humor samples withdrawn from 8 healthy subjects (control), 3 patients suffering from non open angle glaucoma (non POAG) and 18 patients suffering from primary open angle glaucoma (POAG) were analyzed. The aqueous humor samples were withdrawn from the eye anterior chamber of the patient using a syringe according to the conventional practice.

Table 1

| distribution of the clinical-pathological characteristics of the patients and nestin levels (ng) in the aqueous humor samples from each patient | | | |
|---|---|---|---|
| | **Pathology** | | |
| Code | POAG | Calculated value ng | Age (years) |
| UA52 | POAG | 1013.9 | 89 |
| UA 272 | POAG | 545.87 | 90 |
| UA 281 | POAG | 3829 | 85 |
| UA 159 | POAG | 7915.4 | 86 |
| UA 146 | control | -2488.7 | 54 |
| UA 19 | control | -401.38 | 80 |
| UA 10 | control | -7479600 | 86 |
| UA 49 | control | -782.9 | 69 |
| UA 51 | cataract | -4164.2 | 83 |
| UA 227 | POAG | -2782.1 | 76 |
| UA 190 | POAG | 6647.9 | 61 |
| UA 167 | POAG | 1669.7 | 60 |
| UA 147 | POAG | 7221.9 | 81 |
| UA 172 | control | 2955.9 | 87 |
| UA 23 | cataract | -7474300 | 87 |
| UA 40 | POAG | -36853 | 74 |
| UA 76 | glaucoma and CV | 34763 | 90 |
| UA 275 | glaucoma | 11126 | 63 |
| UA 235 | glaucoma POAG | 6086.5 | 76 |
| UA 70 | POAG | 6095.9 | 89 |
| UA 61 | POAG | 1134.3 | 90 |
| UA 229 | POAG | 1133.6 | 82 |
| UA 7 | juvenile glaucoma | -748060 | 73 |
| UA 36 | control | -19205 | 61 |
| UA 23 | POAG | -19183 | 52 |
| UA 40 | initial glaucoma | 1189.8 | 82 |

(continued)

| distribution of the clinical-pathological characteristics of the patients and nestin levels (ng) in the aqueous humor samples from each patient | | | |
|---|---|---|---|
| | **Pathology** | | |
| Code | POAG | Calculated value ng | Age (years) |
| UA 141 | POAG of colour | -2410.7 | 81 |
| UA 121 | POAG | 7007.9 | 60 |
| UA 151 | POAG | 6944.1 | 80 |
| UA 120 | initial POAG | 197.6 | 98 |
| | **Controls** | | |
| Code | Controls | Calculated value ng | Age (years) |
| UA 19 | control | -401.38 | 80 |
| UA 10 | control | -7479600 | 86 |
| UA 49 | control | -782.9 | 69 |
| UA 51 | control | -4164.2 | 83 |
| UA 227 | cataract | -2782.1 | 76 |
| UA 23 | control | -7474300 | 87 |
| UA 40 | cataract | -36853 | 74 |
| UA 3 | control | -19183 | 52 |

Statistical Analysis of the Results

Data were analyzed by appropriate statistical tests such as Kruskal-Wallis test and unpaired t-test.

Table 2: t-test for Pathological QCM Patologici vs Controls

| | Average | t-value | P-value |
|---|---|---|---|
| Control, POAG | -200915.588 | -2.563 | 0.0171* |
| Control, G non POAG | -186909.773 | -0.917 | 0.3831 |
| POAG , G non POAG | 14005.815 | 2.780 | 0.0119* |
| * P <0.05 (statistical significance) | | | |

Table 3: Kruskal -Wallis Test for Pathological QCM vs Controls

| Experimental conditions | 2 |
|---|---|
| Groups | 3 |
| H | 19.857 |
| P-value | <0.0001** |
| ** P<0.001 (statistical significance) | |

The statistical tests which were used are: Kruskal-Wallis test which analyzes non parametric data and the unpaired t-test for parametric data. It can be seen in table 2 that the difference in nestin values between controls and pathologicals, as well as between the subjects with open angle glaucoma and subjects with other types of glaucoma is statistically significative (t-test). In table 3 there is again the statistically significant difference of the presence of nestin protein evaluated by another statistical test (Kruskal -Wallis Test) by comparing pathological subjects vs controls.

Early diagnosis method

The IOP (intraocular pressure) and VFD (visual field defect) values of some patients with POAG and the nestin values were measured and calculated according to the method of the present invention.

The obtained values are the following:

Table 4

| Code | IOP (mmHg) | VDF (GGS) | Nestin (ng) |
|------|-----------|-----------|-------------|
| UA52 | 20.1 | 5 | 1013.9 |
| UA61 | 18.6 | 2 | 1134.3 |
| UA76 | 19.3 | 3 | 34763 |

It can be further noted that, when the intraocular pressure increases in the subject with POAG, the presence of nestin can be seen by QCM and the nestin value increases depending from the advanced stage of the disease. In the plot of Figure 5 it is highlighted that, based on the data reported in Table 4, nestin is correlated to the IOP increase as well as to VDF in the patients suffering from POAG.

**Claims**

1. A functionalized biosensor comprising a gold electrode, a self-assembling molecules layer and an identification layer, said identification layer comprising anti-nestin antibodies, wherein the self-assembling layer comprises bifunctional molecules able to bind to the electrode's gold and to be functionalized to covalently bind the anti-nestin antibody.

2. A method for the early diagnosis of glaucoma comprising the determination of the amount of nestin in an aqueous humor sample from a patient by using a biosensor functionalized with an anti-nestin antibody, and a quartz crystal microbalance.

3. The method according to claim 2 comprising the following steps:

    i. measuring the oscillation frequency of the quartz crystal by quartz crystal microbalance in the presence of the biosensor functionalized with anti-nestin antibodies;
    ii. adding an aqueous humor sample on said biosensor;
    iii. measuring the change of oscillation frequency of the quartz crystal; and
    iv. determining the amount of nestin in the aqueous humor sample.

4. A method according to claim 3 wherein the determination of the amount of nestin is carried out automatically through a software which, when implemented on an electronic device equipped with a detector, is capable to allow the detector to emit a light signal of different color depending on the presence or absence of nestin in the sample.

5. A kit for the early diagnosis of glaucoma comprising the functionalized biosensor according to claim 1 contained into a device with a plastic case and an USB exit which allows the connection with a PC for the recording and management of the detected data.

**Patentansprüche**

1. Funktionalisierter Biosensor, umfassend eine Goldelektrode, eine Schicht mit selbstorganisierenden Molekülen und eine Identifikationsschicht, wobei die Identifikationsschicht Anti-Nestin-Antikörper umfasst, wobei die selbstorganisierende Schicht bifunktionelle Moleküle umfasst, die dazu in der Lage sind, an das Gold der Elektrode zu binden und funktionalisiert zu werden, um den Anti-Nestin-Antikörper kovalent zu binden.

2. Verfahren zur Frühdiagnose von Glaukom, umfassend die Bestimmung der Menge an Nestin in einer Kammerwasserprobe eines Patienten durch Verwenden eines Biosensors funktionalisiert mit einem Anti-Nestin-Antikörper und einer Quarzkristall-Mikrowaage.

3. Verfahren nach Anspruch 2, umfassend die folgenden Schritte:

i. Messen der Oszillationsfrequenz des Quarzkristalls durch Quarzkristall-Mikrowaage in der Gegenwart des Biosensors funktionalisiert mit Anti-Nestin-Antikörpern;
ii. Hinzufügen einer Kammerwasserprobe auf dem Biosensor;
iii. Messen der Änderung der Oszillationsfrequenz des Quarzkristalls; und
iv. Bestimmen der Menge an Nestin in der Kammerwasserprobe.

4. Verfahren nach Anspruch 3, wobei die Bestimmung der Menge an Nestin automatisch durch eine Software durchgeführt wird, die, wenn sie auf einer elektronischen Vorrichtung implementiert wird, die mit einem Detektor ausgestattet ist, in der Lage ist, dem Detektor zu ermöglichen, ein Lichtsignal unterschiedlicher Farbe zu emittieren, abhängig von der Gegenwart oder dem Nichtvorhandensein von Nestin in der Probe.

5. Kit zur Frühdiagnose von Glaukom, umfassend den funktionalisierten Biosensor nach Anspruch 1, der in einer Vorrichtung mit einem Kunststoffgehäuse und einem USB-Ausgang enthalten ist, der die Verbindung mit einem PC zur Aufzeichnung und Verwaltung der erfassten Daten ermöglicht.

**Revendications**

1. Biocapteur fonctionnalisé comprenant une électrode en or, une couche de molécules capable d'auto-assemblage et une couche d'identification, ladite couche d'identification comprenant des anticorps anti-nestine, dans lequel la couche capable d'auto-assemblage comprend des molécules bifonctionnelles capables de se lier à l'or de l'électrode et d'être fonctionnalisées pour se lier par covalence à l'anticorps anti-nestine.

2. Procédé destiné au diagnostic précoce du glaucome comprenant la détermination de la quantité de nestine dans un échantillon d'humeur aqueuse d'un patient en utilisant un biocapteur fonctionnalisé avec un anticorps anti-nestine, et une microbalance à cristal de quartz.

3. Procédé selon la revendication 2 comprenant les étapes suivantes :

i. mesure de la fréquence d'oscillation du cristal de quartz par la microbalance à cristal de quartz en présence du biocapteur fonctionnalisé avec des anticorps anti-nestine ;
ii. ajout d'un échantillon d'humeur aqueuse sur ledit biocapteur ;
iii. mesure du changement de la fréquence d'oscillation du cristal de quartz ; et
iv. détermination de la quantité de nestine dans l'échantillon d'humeur aqueuse.

4. Procédé selon la revendication 3 dans lequel la détermination de la quantité de nestine est réalisée automatiquement à l'aide d'un logiciel qui, quand il est mis en œuvre sur un dispositif électronique équipé d'un détecteur, est capable de permettre au détecteur d'émettre un signal lumineux de couleur différente selon la présence ou l'absence de nestine dans l'échantillon.

5. Trousse destinée au diagnostic précoce du glaucome comprenant le biocapteur fonctionnalisé selon la revendication 1 contenu dans un dispositif avec un boîtier en plastique et une sortie USB qui permet la connexion avec un PC pour l'enregistrement et la gestion des données détectées.

FIG. 1

**Box Plot**
**Grouping Variable(s): Disease**

FIG. 2

**QCM analysis program**

FIG. 3

FIG. 4

FIG. 5

Regression Plot
Row exclusion: nestin database.svd

Y = -2.944 + .288 * X; R^2 = .295

**EP 3 745 130 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2012149809

**Non-patent literature cited in the description**

- **IZZOTTI et al.** Molecular Damage in Glaucoma: from Anterior to Posterior Eye Segment. *The MicroRNA Role,* 2015